# EUROPEAN PATENT SPECIFICATION

(11) **EP 1 121 457 B1**
(45) Date of publication and mention of the grant of the patent: **18.08.2004**
(21) Application number: 99956433.9
(22) Date of filing: 06.10.1999
(51) Int. Cl.: C12Q 1/00, C07K 14/48, A61K 38/27

(54) **USE OF SOCS-2 OR CIS TO SCREEN FOR COMPOUNDS ENHANCING GROWTH HORMONE EFFECT**
VERWENDUNG VON SOCS-2 ODER CIS ZUM 'SCREENEN' VON SUBSTANZEN, DIE EINEN WACHSTUMSHORMONEFFEKT VERSTÄRKEN
UTILISATION DE SOCS-2 OU DE CIS POUR CRIBLER DES COMPOSES RENFOR ANT L'EFFET DE L'HORMONE DE CROISSANCE

(30) Priority: 06.10.1998 SE 9803398
(43) Date of publication of application: 08.08.2001
(73) Proprietor: Sahltech i Göteborg AB, 411 39 Göteborg (SE)
(72) Inventor: NORSTEDT, Gunnar, S-167 75 Bromma (SE); TOLLET EGNELL, Petra, S-178 00 Ekerö (SE); MORALES, Amilcar Flores, S-114 89 Stockholm (SE)
(74) Representative: Bergvall-Eftring, Stina Lena
(86) International application number: PCT/SE1999/001788
(87) International publication number: WO 2000/020624

(56) References cited:
- WO-A1-98/20023
- TIMOTHY E. ADAMS: 'Growth Hormone Preferentially Induces the Rapid, Transient Expression of SOCS-3, a Novel Inhibitor of Cytokine Receptor Signaling' JBC ONLINE vol. 273, no. 3, April 1999, DENMARK, pages 1 - 9, XP002921909

## Description

### Field of the invention

This invention relates to the identification of a cellular mechanism that determines the sensitivity towards a hormonal signal, and a strategy to interfere with the above mentioned cellular mechanism in order to pharmacologically change hormonal sensitivity. More specifically, this invention relates to the provision of a method to increase the cellular sensitivity to growth hormone (GH) by the interference of a so-called SOCS (suppressor of cytokine signaling) signal. Said provision is obtained by the use of SOCS-2 or CIS to screen for compounds which enhance the effects of grow hormone.

### Backeround of the invention

GH is a hormone that is widely used as a therapeutical agent. The most well known medical indication for GH treatment is dwarfism, however, other medical areas where GH treatment might be indicated have been suggested. These areas include certain lipid disorders, heart conditions and disorders of body composition, metabolism and ageing. The presently used GH therapy normally consists of daily injections of a relatively large (198 amino acid) recombinantly manufactured GH. This type of treatment is associated with a relatively high cost and inconvenience for the patient. In light of the expected increase of the medical use for GH, compounds has been searched for that would serve as substitutes for GH. Indeed, such attempts have resulted in the discovery of compounds that increase the pituitary GH production. Another theoretical alternative would be to manufacture a compound that would mimic the cellular mechanism of GH action. In this regard it is relevant to note that the cellular mechanism of GH action is nowadays partly understood (1). A primary step involves binding of GH to its membrane bound receptor, after which receptor homodimerisation occurs. In turn this leads to the activation of the receptor associated kinase JAK2 (janus kinase 2). Once activated, JAK2 is able of stimulating other downstream pathways such as STAT (signal transducer and activator of transcription), MAPK (mitogen activated protein kinase) and IRS-1/PI3 kinase (insulin receptor substrate 1/phosphatidylinositol 3 kinase). GH activation of the JAK2 pathway is transient in nature; e.g. GH activated phosphorylated JAK2, as well as its downstream target STAT5, are only present during approximately 30 minutes in cells stimulated by GH. Interestingly, the duration of the time STATs are activated can be prolonged by the addition of blockers of protein synthesis (2). This supports the concept that proteins might exist that have the capacity to "shut down" the GH receptor. In another research area that involves the action of cytokines such as interferons and interleukins, a new class of proteins, called SOCS (Suppressors Of Cytokine Signaling) (3, 4) were recently discovered. The SOCS proteins are made in response to cytokine stimulation and block then the activated receptor. At least seven different SOCS proteins have been characterized, and computer-based searches have identified more that 30 different proteins that could contain a SOCS motif in their amino acid sequence. A common feature of these well-defined SOCS proteins is the presence of one SH2 domain (Src homology domain 2) and a novel type of SOCS motif. As stated above, the SOCS were discovered when studying the actions of interleukins and interferons. The ability of certain SOCS proteins to block signaling from interferone/interleukin receptors has been shown in experiments using transfection of cells. In a recent study (5), SOCS-3 was reported to be regulated by GH in cultured cells. To a certain extent this finding was anticipated, since the GH receptor is a member of the cytokine receptor family. The recent discovery of the SOCS family of proteins, taken together with their function as proteins that can "turn off" cytokine receptors, is likely to have led many scientists into a contemplation of their potential use as drug targets. However, as stated above, the fact was that many different SOCS exist and the problem to be solved was to find out which of the different SOCS should be used as a drug target in order to influence a specific function of a cytokine.

In the prior art it is also a well-established clinical and experimental finding that GH deficient subjects are extremely sensitive to GH. Upon prolonged GH treatment this extreme GH sensitivity is reduced.

In the prior art it has been shown that GH regulates SOCS expression, Adams TE et al, "Growth Hormone Preferentially Induces the Rapid, Transient Expression of SOCS-3, a novel inhibitor of cytokine receptor signaling", 1999, vol 273(3), p. 1285ff. This publication contains no information of SOCS levels in GH hypersensitive states. The choice of model cell systems and the particular design of an animal experiment in this publication does not adequately approach the question of hypersensitivity towards GH. This explains that the conclusions drawn are somewhat different in the work of Adams et al compared to what is the basis for the present invention.

In WO 98/20023 different SOCS sequences have been described as is the use of SOCS to modify cytokine functions; little reference is given to GH in this application and there is no reference to individual SOCS and states of GH sensitivity.

### The present invention

We have found that the mechanism which causes this high GH sensitivity is a useful target for drug interference. Such a drug would cause the cell to become more sensitive to an endogenous GH signal. Based on a substantial research effort in the area of GH signaling, we have to our surprise found two molecular targets that can determine GH sensitivity. These targets, defined as SOCS-2 and CIS (cytokine induced SH2-containing protein), have been found to be selectively reduced in conditions of GH deficiency. We have also found that these SOCS proteins are GH regulated. The GH deficient state itself does not represent a severe medical threat to an individual, which leads to the realisation that SOCS-2 and CIS can be reduced in an organism without any severe medical consequences.

The principle underlying the present invention is that a reduction of SOCS-2 and/or CIS will increase the cellular sensitivity to GH. The finding that the above mentioned SOCS are reduced in a situation only associated with growth failure, and not with other severe malfunction of the body, indicates a certain specificity for GH in the SOCS-2/CIS system. In turn, this suggests that SOCS-2 and/or CIS could be useful targets to find GH sensitising agents. In the present invention, a cell system is described that could be used to screen for inhibitors of SOCS-2 and CIS. Alternative screening strategies are also exemplified. A therapeutical use of compounds that reduce the levels of SOCS-2 and/or CIS would lead to an increased sensitivity to GH. Such a compound might find its use in patients that suffer from syndromes of GH resistance or insufficient levels of GH. The terminology; reduction of the level of SOCS-2 and/or CIS, refers to a reduction of cellular SOCS-2 and/or CIS protein content either by an interference with (i) the synthetic machinery e.g. by reduction of the corresponding SOCS mRNAs or (ii) mechanisms that regulate protein turn-over. In addition reduction of SOCS-2 and CIS could also refer to a reduction of biological activity of SOCS-2 and CIS by e.g. an interference with key functional domains in the proteins.

### Descrintion of the invention

The invention relates to the use of SOCS-2 and/or CIS to screen for compounds which enhance the effects of growth hormone. SOCS-2 and/or CIS, according to the invention, are used as targets in the screening of GH sensitisers. Such a sensitiser, is a compound that either reduces the cellular content of SOCS-2 and/or CIS, or serve as a blocker of the interaction of SOCS-2 and/or CIS with key signaling components of the GH transduction pathway.

The invention also relates to the monitoring of SOCS-2 and/or CIS and further includes the use of SOCS-2 and/or CIS in a conventional assay system to screen for compounds that reduce SOCS-2 and/or CIS comprising cultured cells in which levels (protein and/or mRNA) of SOCS-2 and/or CIS can be monitored. The invention also comprises the use of an assay system to screen for compounds that reduce the biological activity of SOCS-2 and/or CIS comprising purified or recombinant SOCS-2 and/or CIS and proteins, optionally isolated, that link SOCS-2 and/or CIS to signaling molecules dependent on GH.

A further embodiment relates to a method of screening for compounds that reduces SOCS-2 and/or CIS comprising cultured cells in which levels of SOCS-2 and/or CIS can be monitored and yet another to a method to screen for compounds that reduce the biological activity of SOCS-2 and/or CIS comprising purified or recombinant SOCS-2 and/or CIS, and proteins, optionally isolated, that link SOCS-2 and/or CIS to signaling molecules dependent on GH. The invention also comprises a method to increase cellular sensitivity to GH by reduction of the levels of SOCS-2 and/or CIS.

We have identified that SOCS-2 and/or CIS serve as useful targets to find GH sensitisers. We claim that a compound, that would either reduce the cellular content of SOCS-2 and/or CIS, or serve as a blocker of the interaction of SOCS-2 and/or CIS with key signaling components of the GH transduction pathway, will sensitise for GH. In the present invention we also provide strategies to find such compounds.

Materials and methods as used according to the invention are in line with established techniques. The invention encompasses all embodiments as disclosed in the claims.

### Examples of the invention

The examples as provided demonstrate that a cellular reduction in SOCS-2 and CIS levels is associated with a GH hypersensitive state, and that GH regulates the expression of SOCS-2 as well as CIS. An example is also provided of a cell system according to which compounds can be found that reduce the functions of SOCS-2 as well as CIS. Finally, we outline a strategy to design an in vitro assay to identify a compound that inhibits the interaction of SOCS-2 as well as CIS with signaling components of the GH transduction pathway. These examples are used only to illustrate the invention and are not intended to limit the scope of the invention.

### Example 1

### A GH hypersensitive state is associated with a reduction of SOCS-2 mRNA and CIS mRNA

Chronically (2 weeks) adult hypophysectomized (HX) male and female rats were compared to intact controls. A selection of different tissues (all known to be GH responsive) consisted of liver, muscle and adipose tissue. From these tissues RNA were prepared and levels of SOCS-2 mRNA, CIS mRNA and SOCS-3 mRNA were analysed using a RNAse protection/solution hybridisation assay (6). The probes that were used to detect these transcripts were isolated from rat genomic DNA using PCR technique with the following primers:
SOCS-3 Forward primer: 5' GAG TAC CCC CAA GAG AGC TTA CTA C 3'
Reverse Primer: 5' CTC CTT AAA GTG GAG CAT CAT ACT G 3'
PCR product length: 209 bases

SOCS-2 Forward Primer: 5' GAG CTC AGT CAA ACA GGA TGG TAC T 3'
Reverse Primer: 5' AGA ATC CAA TCT GAA TTT CCC ATC T 3'
PCR product length: 201 bases

CIS: Forward Primer: 5' ATC TTG TCC TTT GCT GGC TGT 3'
Reverse Primer: 5' CCC GAA GGT AGG AGA ACG TCT 3'
PCR product length: 215 bases

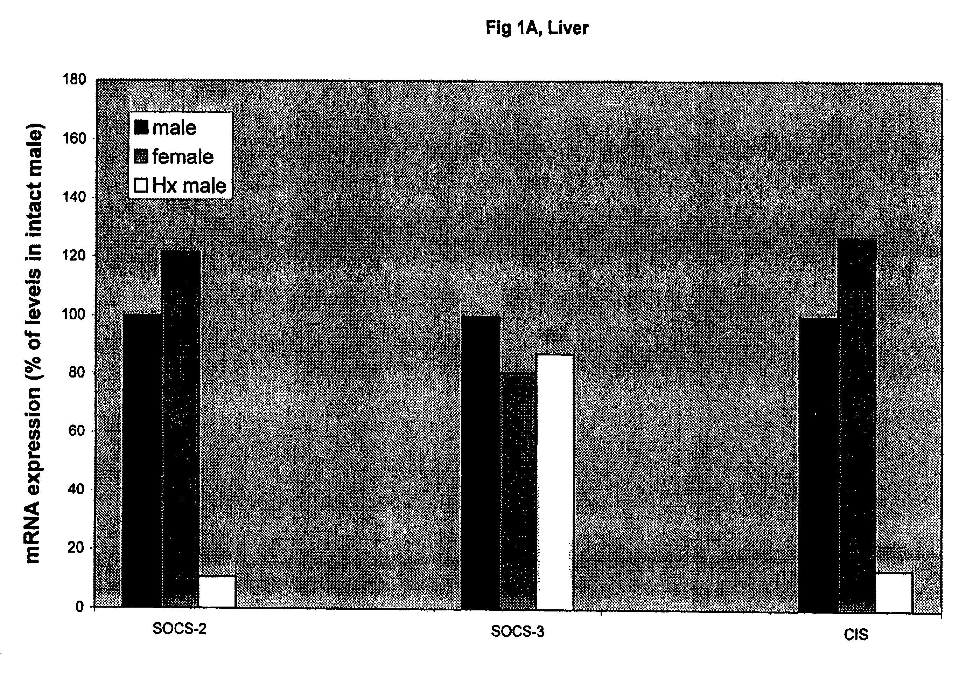

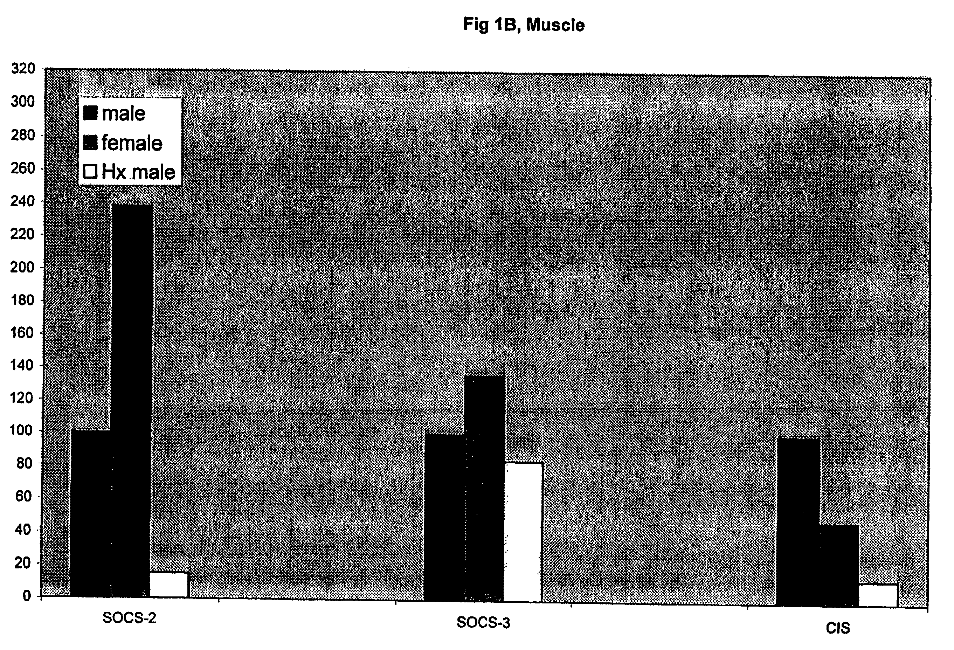

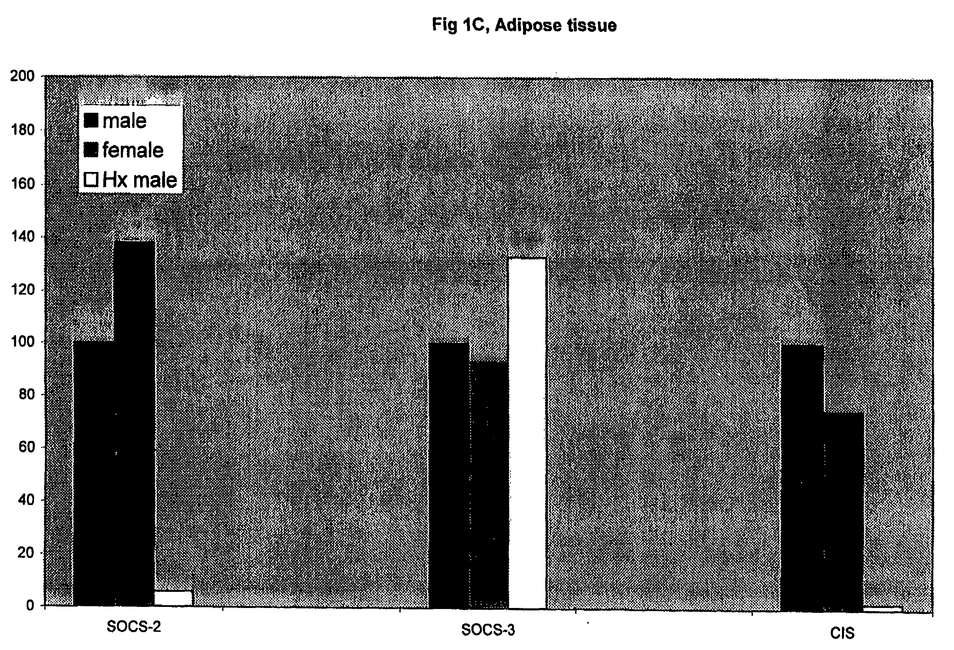

The PCR products were cloned into a plasmid vector containing T3/T7 promoter.
In this experiment, hypophysectomy caused the expected growth failure, but in other aspects the animals were in good condition. As can be seen in Fig 1, hypophysectomy caused a dramatic reduction in transcript levels of SOCS-2 and CIS, whereas the expression of SOCS-3 was not altered. This was the situation in liver (Fig 1A), muscle (Fig1B) and fat (Fig1C). The marked reduction of these two SOCS transcripts, taken together with the previously established SOCS function (to block cytokine receptors such as the GH receptor) and the well known state of GH increased GH sensitivity in hypophysectomised rats, allows one to draw the conclusion that SOCS-2 and/or CIS could be a major determinant of GH sensitivity in different tissues.

### Example 2

### GH regulates the expression of SOCS-2

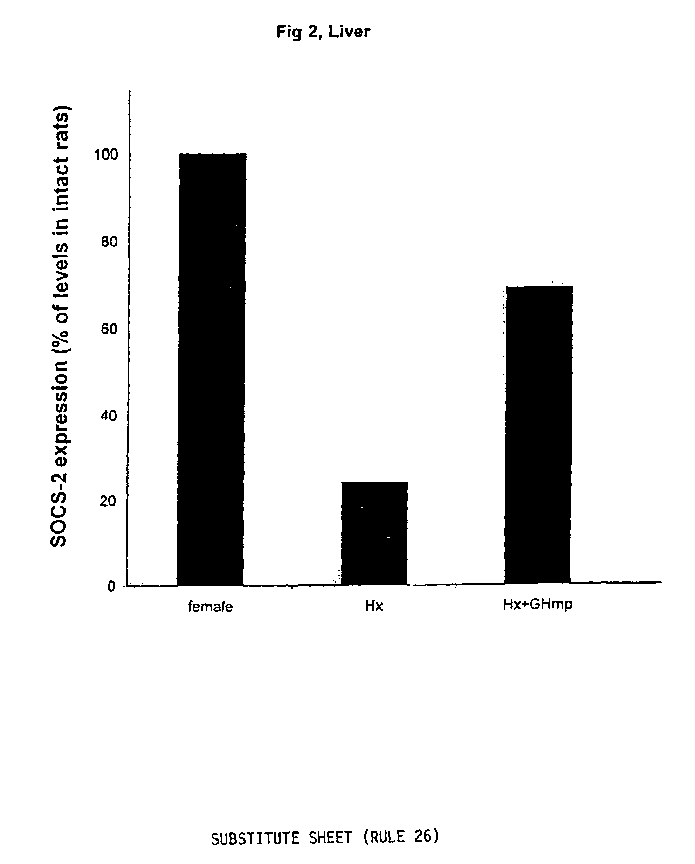

In a follow up experiment, GH (hGH 5 ug/h/250 mg BW) was infused into hypophysectomised (HX) rats for one week using osmotic minipumps. The experiment also included a group of intact female rats and a group of HX rats, each group consisted of four animals and SOCS-2 mRNA was analysed in RNA prepared from liver. As shown in Fig 2, GH treatment caused a partial restoration of the SOCS-2 mRNA level, indicating that SOCS-2 transcripts are GH regulated. These data fit well with a concept of SOCS proteins being GH receptor "switch off" signals, and the known situation in which a continued GH treatment reduce GH sensitivity.

### Example 3

### A cell system to be used to interfere with functions of SOCS-2 and CIS

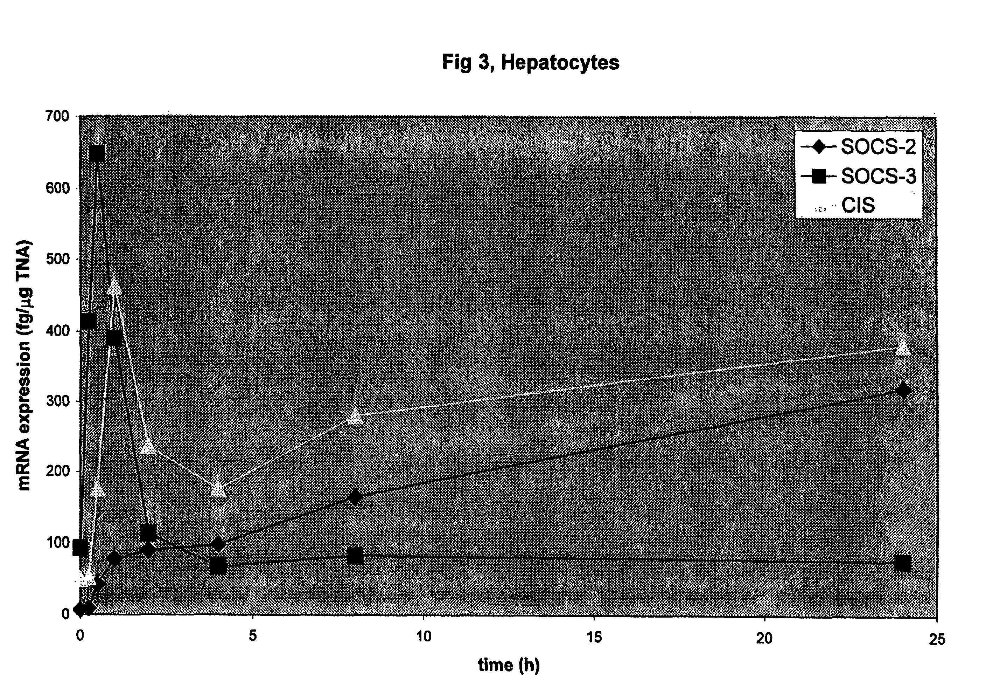

Rat liver hepatocytes were isolated and cultured on matrigel in serum free Williams E medium. Hormonal treatments were started 40 hours after seeding. In a time course study, SOCS-2, SOCS-3 and CIS mRNA were measured and the results are shown in Fig 3. From the data it is clear that all of the different SOCS transcripts were increase by GH. The kinetics were however different; SOCS-3 was only transiently increased by GH, whereas SOCS-2 and CIS mRNA responded to GH treatment by a continued increase for the duration of the experiment. Taken together with the results from experiment 1, these data imply that SOCS-2 and CIS are the prime candidates to determine GH sensitivity. It is also demonstrated that SOCS-2 and CIS were detectable in a situation without GH stimulus. For this reason it would be possible to use unstimulated cells in a drug screening experiment to search for compounds that reduce the levels of SOCS-2 and/or CIS. In such an experiment an assay could be based on the detection of SOCS-2 mRNA and/or CIS mRNA using conventional means (RNA/RNA hybrid formations or DNA/RNA hybrid formations) for mRNA detection. Alternatively, the SOCS-2 and/or CIS protein could be detected by immunological techniques or by genetically engineer a cell to express a tagged SOCS-2/CIS protein.

It should also to be noted that other types of tissues, such as muscle and adipose tissue, responded in a similar manner as liver to GH deficiency and treatment (Fig 1 B and C). Subsequently, it should likewise be possible to develop a screening assay using cells derived from muscle or adipose tissue. Alternatively, any cell-line that express GH receptors and SOCS-2 and/or CIS could be used. In yet another embodiment a screening strategy could be designed in cells that uses the combination of GH addition and SOCS-2/CIS reduction. The end point measuring parameters would then measure both facilitation of a GH signal, in the form of e.g. a proliferative response, a metabolic alteration or a reporter gene activation in combination with the above mentioned way to measure a SOCS-2/CIS reduction.

### Example 4

### In vitro assays to identify a compound that inhibits the interaction of SOCS-2 and/or CIS with signaling components of the GH signal transduction pathway.

SOCS-2 and/or CIS is likely to physically bind to an activated GH receptor-JAK2 complex, either in a direct manner or via an as yet unknown bridging molecule. An activated GH receptor-JAK2 complex can be obtained in several different ways; by immunoprecipitation of membrane extracts from normal cells or tissues, or from cells that have been genetically engineered to express the GH receptor-JAK2 complex. Alternatively, relevant parts of the GH receptor and the JAK2 proteins can be recombinantly expressed and purified. An in vitro assay would then be designed to first establish an assay that would detect the binding between the GH receptor-JAK complex and the preferably recombinantly expressed SOCS-2/CIS protein. For this purpose an easy detection system should be available for at least one of the partners in the complex; such a detection system could be based on antibodies, or on "tagging" one of the proteins with a marker molecule or radioactivity. The subsequent use of this assay would be to screen a compound-collection for substances that would block the interaction between the GH receptor-JAK2 complex and SOCS-2/CIS.

### References:

1. Wood TJ, Haldosén L-A, Sliva D, Sundström M and Norstedt G Stimulation of kinase cascades by growth hormone; a paradigm for cytokine signaling. In; Progress in Nucleic Acid Research and Molecular Biology, 57: 73-94 (1997)
2. Fernandez L, Flores-Morales A, Lahuna O, Sliva D, Norstedt G, Haldosén L-A, Mode A, and Gustafsson J-Å Desenitization of the growth hormone-induced Janus kinase 2 (JAK2)/signal transducer and activator of transcription 5 (Stat5)-signaling pathway requires protein synthesis and phospholipase C. Endocrinology 139: 1815-1824 (1998)
3. Starr R, Willson T A, Viney E M, Murray L J L, Rayner J R, Jenkins B J, Gonda T J, Alexander W S, Metcalf D, Nicola N A and Hilton D J A family of cytokine-inducible inhibitors of signaling Nature, 387: 917-921 (1997)
4. Endo T A, Masuhara M, Yokouchi M, Suzuki R, Sakamoto H, Mitsui K, Matsumoto A, Tanimura S, Ohtsubo M, Misawa H, Miyazaki T, Leonor N, Taniguchi T, Fujita T, Kanakura Y, Komiya S and Yoshimura A A new protein containing SH2 domain that inhibits JAK kinases Nature, 387: 921-924 (1997)
5. Adams T E, Hansen J A, Starr R, Nicola N A, Hilton D J, and Billestrup N Growth hormone preferentially induces the rapid, transient expression of SOCS-3, a novel inhibitor of cytokine receptor signaling J Biol Chem 273: 1285-1287 (1998)
6. Möller C, Arner P, Sonnenfeld T, Norstedt G: Quantitative comparision of insulin-like growth factor I (IGF-I) and IGF-II messenger RNA levels in human and rat tissues analysed by a solution hybridization assay. J. Molecular Endocrinology, 7:213, 1991.

## Claims

1. The use of SOCS-2 (suppressor of cytokine signaling) and/or CIS (cytokine-induced SH2-containing protein), to screen for compounds that reduce the cellular expression of SOCS-2 and/or CIS and thereby enhance the effects of growth hormone.

2. The use according to claim 1 of SOCS-2 in purified or recombinant form.

3. The use according to claim 1 of CIS in purified or recombinant form.

4. The use according to claim 1 of SOCS-2 and/or CIS in a conventional screening assay system.

5. The use according to claim 4 in an assay system comprising cultured cells in which levels of SOCS-2 and CIS can be monitored.

6. The use according to claim 4 in an assay system comprising purified or recombinant SOCS-2 and/or CIS.

7. A method to screen for compounds that enhance the effects of growth hormone by bringing cultured cells into contact with said compounds and assessing the cellular expression of SOCS-2 and/or CIS in said cultured cells.

8. The method according to claim 7 wherein the cellular expression of SOCS-2 and/or CIS is assessed by measuring the content of the proteins SOCS-2 and/or CIS of the cultured cells.

9. The method according to claim 7 wherein the cellular expression of SOCS-2 and/or CIS is assessed by measuring the content of the SOCS-2 mRNA and/or CIS mRNA of the cultured cells.

10. The method according to any of the claims 7-9 also comprising adding GH (growth hormone) to the cultured cells and measuring a GH signal.

11. The method according to claim 10 wherein the GH signal is a proliferative reponse, a metabolic alteration or a reporter gene activation.

12. A method according to any of the claims 7-11 wherein the cultured cells are derived from liver, muscle or adipose tissue.

13. A method to screen for compounds that enhance the effects of growth hormone comprising the steps of
bringing a preparation comprising GH receptor-JAK2 complex into contact with SOCS-2 and/or CIS;
detecting the binding of the GH receptor-JAK2 complex with SOCS-2 and/or CIS in the preparation;
bringing the preparation into contact with the compounds to be screened; and
assessing the blocking of the interaction of SOCS-2 and/or CIS and the GH receptor-JAK2 complex.

14. The method according to claim 13, wherein the SOCS-2 and/or CIS are recombinantly expresssed proteins.

## Patentansprüche

1. Die Verwendung von SOCS-2 (Suppressor der Zytokinsignalvermittlung) und/oder CIS (Zytokin-induziertes SH2-enthaltendes Protein) zur Untersuchung von Verbindungen, die die zelluläre Expression von SOCS-2 und/oder CIS verringern und dadurch die Wirkungen des Wachstumshormons verstärken.

2. Die Verwendung von SOCS-2 in aufgereinigter oder rekombinanter Form gemäß Anspruch 1.

3. Die Verwendung von CIS in aufgereinigter oder rekombinanter Form gemäß Anspruch 1.

4. Die Verwendung von SOCS-2 und/oder CIS in einem konventionellen Screening-Assay-System gemäß Anspruch 1.

5. Die Verwendung gemäß Anspruch 4 in einem Assay-System, das kultivierte Zellen umfasst, in denen die Mengen an SOCS-2 und CIS überwacht werden können.

6. Die Verwendung gemäß Anspruch 4 in einem Assay-System, das aufgereinigte oder rekombinante SOCS-2 und/oder CIS umfasst.

7. Ein Verfahren zur Untersuchung von Verbindungen, die die Wirkungen des Wachstumshormons verstärken, durch das Inkontaktbringen kultivierter Zellen mit besagten Verbindungen und die Beurteilung der zellulären Expression des SOCS-2 und/oder CIS in besagten kultivierten Zellen.

8. Das Verfahren gemäß Anspruch 7, worin die zelluläre Expression von SOCS-2 und/oder CIS durch die Messung des Gehalts der Proteine SOCS-2 und/oder CIS der kultivierten Zellen untersucht wird.

9. Das Verfahren gemäß Anspruch 7, worin die zelluläre Expression von SOCS-2 und/oder CIS durch Messung des Gehalts der SOCS-2 mRNA und/oder CIS mRNA der kultivierten Zellen untersucht wird.

10. Das Verfahren gemäß einem der Ansprüche 7 - 9, das zusätzlich die Zugabe von GH (Wachstumshormon) zu den kultivierten Zellen und das Messen eines GH-Signals umfasst.

11. Das Verfahren gemäß Anspruch 10, worin das GH-Signal eine proliferative Reaktion, eine metabolische Änderung oder eine Reportergenaktivierung ist.

12. Ein Verfahren gemäß einem der Ansprüche 7 - 11, worin die kultivierten Zellen aus Leber-, Muskel- oder Fettgewebe abgeleitet sind.

13. Ein Verfahren zur Untersuchung von Verbindungen, die die Wirkungen des Wachstumshormons verstärken, das die folgenden Schritte umfasst:
das Inkontaktbringen einer einen GH-Rezeptor-JAK2-Komplex umfassenden Zubereitung mit SOCS-2 und/oder CIS;
den Nachweis der Bindung des GH-Rezeptor-JAK2-Komplexes mit SOCS-2 und/oder CIS in der Zubereitung;
das Inkontaktbringen der Zubereitung mit den zu untersuchenden Verbindungen; und
die Beurteilung der Blockierung der Wechselwirkung von SOCS-2 und/oder CIS und dem GH-Rezeptor-JAK2-Komplex.

14. Das Verfahren gemäß Anspruch 13, worin das SOCS-2 und/oder CIS rekombinant exprimierte Proteine sind.

## Revendications

1. L'utilisation de SOCS-2 (suppresseur de signalisation des cytokines) et/ou de CIS (protéine contenant SH2 induite par une cytokine) pour cribler des composés qui réduisent l'expression cellulaire de SOCS-2 et/ou de CIS et ainsi amplifient les effets de l'hormone de croissance.

2. L'utilisation selon la revendication 1 de SOCS-2 sous une forme purifiée ou recombinante.

3. L'utilisation selon la revendication 1 de CIS sous une forme purifiée ou recombinante.

4. L'utilisation selon la revendication 1 de SOCS-2 et/ou de CIS dans un test de criblage classique.

5. L'utilisation selon la revendication 4 dans un système d'essai comprenant des cellules cultivées dans lequel les taux de SOCS-2 et de CIS peuvent être contrôlés.

6. L'utilisation selon la revendication 4 dans un système de test comprenant du SOCS-2 et/ou du CIS purifiés ou recombinants.

7. Procédé de criblage de composés qui amplifient les effets de l'hormone de croissance par mise en contact de cellules cultivées avec lesdits composés et évaluation de l'expression cellulaire de SOCS-2 et/ou de CIS dans lesdites cellules cultivées.

8. Le procédé selon la revendication 7, dans lequel l'expression cellulaire de SOCS-2 et/ou de CIS est évaluée par mesure de la teneur des cellules cultivées en protéines SOCS-2 et/ou CIS.

9. Le procédé selon la revendication 7, dans lequel l'expression cellulaire de SOCS-2 et/ou de CIS est évaluée par mesure de la teneur en ARNm de SOCS-2 et/ou en ARNm de CIS des cellules cultivées.

10. Le procédé selon l'une quelconque des revendications 7 à 9, comprenant aussi l'addition de GH (hormone de croissance) aux cellules cultivées et la mesure d'un signal GH.

11. Le procédé selon la revendication 10, dans lequel le signal GH est une réponse proliférative, une altération métabolique ou une activation d'un gène reporter.

12. Le procédé selon l'une quelconque des revendications 7 à 11, dans lequel les cellules cultivées proviennent du foie, de muscles ou de tissus adipeux.

13. Procédé de criblage de composés qui amplifient les effets de l'hormone de croissance, qui comprend les étapes de :
- mise en contact d'une préparation comprenant un complexe récepteur de GH-JAK2 avec SOCS-2 et/ou CIS ;
- détection de la liaison du complexe récepteur de GH-JAK2 avec SOCS-2 et/ou CIS dans la préparation ;
- mise en contact de la préparation avec les composés à cribler ; et
- évaluation du blocage de l'interaction de SOCS-2 et/ou de CIS et du complexe récepteur de GH-JAK2.

14. Le procédé selon la revendication 13, dans lequel le SOCS-2 et/ou le CIS sont des protéines exprimées par une technique recombinante.
